(19) Europäisches Patentamt
European Patent Office
Office européen des brevets

(11) Veröffentlichungsnummer: **0 002 217**
**B1**

(12) # EUROPÄISCHE PATENTSCHRIFT

(21) Anmeldenummer: **78101399.0**

(22) Anmeldetag: **18.11.78**

(51) Int. Cl.³: **C 07 D 239/30**

(54) **Verfahren zur Herstellung von Tetrachlorpyrimidin**

(30) Priorität: **29.11.77 DE 2753204**

(43) Veröffentlichungstag der Anmeldung:
**13.06.79 Patentblatt 79/12**

(45) Bekanntmachung des Hinweises auf die Patenterteilung:
**10.12.80 Patentblatt 80/25**

(84) Benannte Vertragsstaaten:
**BE CH DE FR GB**

(56) Entgegenhaltungen:
**DE - B - 1 094 737**
**Angewandte Chemie, 82.Jahrgang, (1970), Nr.2**
**Verlag Chemie GmbH, R. Braden et al.**
**"Direktsynthese chlorierter Pyrimidine",**
**Seiten 78,79**

(73) Patentinhaber: **BAYER AG**
**Zentralbereich Patente, Marken und Lizenzen**
**D - 5090 Leverkusen 1, Bayerwerk (DE)**

(72) Erfinder: **Beck, Gunther, Dr.**
**Am Mittelberg 19**
**D - 5090 Leverkusen (DE)**
**Dankert, Gerhard, Dr.**
**Arthur-Hantzsch-Strasse 44**
**D - 5000 Köln 80 (DE)**
**Döring, Fritz**
**Am Hang 13**
**D - 5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 217 B1

# Verfahren zur Herstellung von Tetrachlorpyrimidin

Gegenstand der vorliegenden Erfindung is ein neues Verfahren zur Herstellung von Tetrachlorpyrimidin.

Das Verfahren ist dadurch gekennzeichnet, daß man (2-Cyanäthyl)-isocyaniddichlorid der Formel

$$NC - CH_2 - CH_2 - N = CCl_2 \qquad (I)$$

in der Gasphase im Temperaturbereich von 200 bis 650°C mit Chlor umsetzt.

Die besten Ausbeuten erhält man dabei im allgemeinen, wenn man mit 3 Mol Chlor pro Mol (I) umsetzt. Setzt man mit geringeren Mengen Chlor um, erhält man ebenfalls Tetrachlorpyrimidin, aber in geringeren Ausbeuten. Um sicherzustellen, daß 3 Mol Chlor pro Mol (I) umgesetzt werden, setzt man in praxi mindestens 3 Mol, im allgemeinen 3 — 4,5 Mol Chlor pro Mol (I), also einen bis zu 50% igen Überschuß, ein. Ein noch größerer Chlorüberschuß kann zwar ohne Ausbeuteminderung angewandt werden, ist jodoch unökonomisch.

Für die Durchführung des erfindungsgemaßen Verfahrens hat es sich als zwechmäßig erwiesen, in Gegenwart von Chlorierungskatalysatoren zu arbeiten. Beispielhaft seien Siliciumdioxid (Silikagel) und Bims, welche mit $CuCl_2$ oder mit $FeCl_3$imprägniert sind, genannt. Besonders bevorzugt als Katalysator sind jedoch die üblichen Aktivkohlen, wie sie beispielsweise in Ullmanns Encyklopädie der technischen Chemie, Band 9, Seite 806 beschrieben sind.

Die Menge an Katalysator wird vorzugsweise derart bemessen, daß pro Gramm stündlich eingebrachtem (I) 0,05 — 10 ml, vorzugsweise 0,1 — 5 ml zur Verfügung stehen.

Das erfindungsgemäße Verfahren wird nach den für Gasphasenchlorierungen üblichen Techniken durchgeführt. Vorzugsweise wird die exotherme Reaktion in einem senkrecht stehenden Strömungsrohr durchgeführt, wobei die entstehende Wärme durch ein umlaufendes Inertgas ($N_2$, $CO_2$), durch eine umlaufende Badflüssigkeit oder aber auch mit einem Wirbelbett z.B. aus Korund abgeführt wird.

Ebenso ist zur Wärmeabfuhr die Verdünnung des Reaktionsgemisches beispielsweise mit Stickstoff, Chlorwasserstoff, Tetrachlorkohlenstoff oder Tetrachlorpyrimidin von Vorteil.

Das Wort Strömungsrohr ist im vorliegenden Fall breit auszulegen. Es kann sich um rohrartige Reaktionsräume verschiedener Ausführung handeln, z.B.: geradlinig, spiralförmig oder gebogen, in denen die Ümsetzung mit unterschiedlicher Geschwindigkeit stattfinden kann; hierunter fallen auch wirbelschichtartige Anordnungen. Die Strömungsgeschwindigkeit ist von der Temperature und Verdünnung des Reaktionsgemisches abhängig.

Zur Durchführung des erfindungsgemäßen Verfahrens bringt man (I) und Chlor sowie gegebenenfalls eines der oben genannten Verdünnungsmittel zunächst in einen als Verdampfer fungierenden, auf etwa 200 — 260°C vorerhitzten Kolben, aus dem dann über eine ebenfalls auf 200 — 260°C oder höher erhitzte Brücke die dampfförmigen Reaktionspartner in das oben geschilderte, mit dem Katalysator gefüllte Strömungsrohr eintreten, in welchem die exotherm verlaufende Umsetzung zu Tetrachlorpyrimidin stattfindet. Der Temperaturbereich kann dabei zwischen 200 und 650°C, vorzugsweise 200 bis 500°C, variieren.

Das aus dem Strömungsrohr austretende gasförmige Tetrachlorpyrimidin wird im allgemeinen zunächst kondensiert und nach beendeter Reaktion bei leicht erhöhten Temperaturen (etwa 50°C) durch kurzes Anlegen von Wasserstrahlvakuum entgast. Die gaschromatographisch ermittelte Reinheit des Tetrachlorpyrimidins liegt im allgemeinen bei $\geqq$ 95%. Falls erwünscht, läßt sich durch Fraktionierung an einer Kolonne ($Kp_{12}$ 108 — 110°C) leicht ein gaschromatographisch mindestens 99 %iges Tetrachlorpyrimidin gewinnen.

Die Chlorierung kann selbstverständlich auch kontinuierlich erfolgen.

Tetrachlorpyrimidin ist als Reaktivkomponente für die Herstellung von Reaktivfarbstoffen geeignet (vergl. z.B. Belgische Patentschrift Nr. 578 933). Darüber hinaus findet Tetrachlorpyrimidin Anwendung als fungizides und sporizides Mittel (US-Patentschrift Nr. 3 227 612).

Das als Ausgangsprodukt verwendete (2-Cyanäthyl)-isocyaniddichlorid der Formel (I) ist neu. Es wird folgendermaßen hergestellt:

Das in bekannter Weise (Französische Patentschrift 976 959) z.B. aus Formamid und Acrylnitril zugängliche (2-Cyanäthyl)-formamid (II) wird gemaß der Reaktionsgleichung

$$\underset{\text{(II)}}{NC - CH_2 - CH_2 - NH - CHO} + SOCl_2 + Cl_2 \rightarrow$$

$$\underset{\text{(I)}}{NC - CH_2 - CH_2 - N = CCl_2} + SO_2 + 2\ HCl$$

bei Temperaturen von 40 — 80°C, vorzugsweise 50 — 75°C, mit Thionylchlorid und Chlor, bzw. einer unter den Reaktionsbedingungen Chlor abspaltenden Verbindung, vorzugsweise Sulfurylchlorid, umgesetzt.

Besonders gute Ausbeuten (etwa 90%) und hohe Reinheiten (bis zu 98%) an (I) erhält man, wenn man (2-Cyanäthyl)-formamid(II) in eine Mischung aus Thionylchlorid und Sulfurylchlorid bzw. Chlor einträgt, bei der das molare Verhältnis Thionylchlorid: Sulfurylchlorid bzw. Chlor wesentlich größer als 1 : 1, im allgemeinen etwa 3 : 1 bis 10 : 1 beträgt. Ein noch höheres Molverhältnis ist möglich, bringt aber keine wesentlichen Vorteile.

Das Molverhältnis Chlor bzw. Chlor abspaltende Verbindung, insbesondere Sulfurylchlorid, zu (II) sollte zur Erzielung einer möglichst hohen Ausbeute an (I) mindestens 1 : 1 betragen. In der Praxis wendet man Molverhältnisse zwischen 1 : 1 und 2,5 : 1 an. Ein noch höheres Molverhältnis ist möglich, bringt aber keine Vorteile.

Es kann zweckmäßig sein, das (2-Cyanäthyl)-formamid (II) bei Temperaturen in das Chlorierungsgemisch einzutragen, die unterhalb 40 — 80°C, vorzugsweise 50 — 75°C, liegen, beispielsweise zwischen 20 und 35°C, und das Reaktionsgemisch erst dann aufzuheizen, wenn nach erfolgter Zugabe von (II) die erste, leicht exotherme Reaktion abgeklungen ist.

Die Hauptreaktion findet zwischen 50 und 75°C statt und ist im allgemeinen nach spätestens zwei Studen beendet.

Überschüssiges Chlorierungsmittel, insbesondere das überschüssige Thionylchlorid, kann nach dem Abdestillieren vom wesentliche schwerer flüchtigen (2-Cyanäthyl)-isocyaniddichlorid (I) ($Kp_{13}$ 107°C) für weitere Ansätze verwendet werden. Selvstverständlich kann man auch kontinuierlich arbeiten.

Das nach dem Abziehen des überschüssigen Thionylchlorids und gegebenenfalls überschüssigen Chlorierungsmittels, insbesondere Sulfurylchlorid, hinterbleibende (2-Cyanäthyl)-isocyaniddichlorid (I) ist im allgemeinen bereits von hoher Reinheit (>90%) und kann direkt in die oben beschriebene Gasphasenchlorierung eingesetzt werden. Durch eine einfache Destillation, bei der nur geringfügige Mengen undestillierbarer Rückstände hinterbleiben, erhält man bei $Kp_{13}$ 107°C ein gaschromatographisch fast 98% reines (I) in etwa 90%iger Ausbeute.

In angewandte Chemie, 82. Jahrgang, Nr. 2, Seiten 78 — 79, wird die Umsetzung von $\alpha$-dihalogenierten Isocyanid-dichloriden mit Nitrilen zu chlorierten Pyrimidinen beschrieben, wobei die Nitrile in $\alpha$-Stellung zwei Wasserstoffatome besitzen müssen und im Gegenwart von Lewis-Säure gearbeitet werden muß. Dieses Verfahren — bei dem im übrigen die im Vergleich zu 2-Cyanethylisocyaniddichlorid wesentlich schwieriger zugänglichen $\alpha$-dihalogenierten Isocyaniddichloride eingesetzt werden müssen — ist grundsätzlich verschieden bon dem beanspruchten Verfahren und legt dieses in keiner Weise nahe.

## Beispiel 1

In einen als Verdampfer fungierenden 500 ml-Dreihalskolben, der durch ein Ölbad von 240°C beheizt ist und mit einer durch eine elektrische Heizbandage auf 240°C beheizten Brücke versehen ist, werden im Verlauf von 95 Minuten gleichzeitig 123 g (0,815 Mol) 2-Cyanäthyl)-isocyaniddichlorid und 231 g (3,25 Mol) Chlor mittels Dosierungspumpe bzw. Strömungsmesser eingebracht. An die Brücke schließt sich ein vertikal aufgehängtes, durch elektrische Wicklung auf 240°C beheiztes Strömungsrohr (Länge 300 mm, Durchmesser 30 mm) an, das mit Aktivkohle von einem Teilchendurchmesser von 2 — 3 mm gefüllt ist (Kontaktvolumen 185 ml) und in dem das im Verdampfer erzeugte Gasgemisch zur Umsetzung gelangt. Zur Abfuhr der Reaktionswärme ist das Strömungsrohr nach Art eines Liebigkühlers von einem Außenmantel umgeben, durch den Inertgas geleitet wird.

Ein konaxial in das Strömungsrohr eingebrachtes Thermoelement kontrolliert die Kontakttemperatur von 240°C. Als Vorlage lient ein durch ein Eis-Wasser-Bad gekühlter 2 1-Zweihalskolben, in dem das Reaktionsprodukt kondensiert wird und durch dessen zweiten Stutzen der gebildete Chlorwasserstoff und gegebenenfalls überschüssiges Chlor abgeleitet werden.

Nach beendeter Reaktion wird das Kondensat bei 50°C im Wasserstrahlvakuum kurz entgast. Auswaage: 175 g. Die gaschromatographische Analyse ergab einen Gehalt von 96% (entsprechend 168 g) Tetrachlorpyrimidin; das entspricht einer Ausbeute von 94,6% der Theorie.

## Beispiel 2

In der Apparatur des Beispiels 1 werden im Verlauf von 50 Minuten 130 g (0,86 Mol) (2-Cyanäthyl)-isocyaniddichlorid in Gegenwart von 244 g (3,44 Mol) Chlor bei 240°C verdampft und durch das Strömungsrohr geleitet. Durch den Außenmantel des Strömungsrohres wird Inertgas in dem Maße geleitet, daß sich der Kontakttemperatur zwischen 240 und 260°C hält.

Nach Aufarbeitung analog Beispiel 1 elhält man 180 g Kondensat mit einem gaschromatographisch ermittelten Gehalt von 95,9% (entsprechend 172,6 g) Tetrachlorpyrimindin; das entspricht einer Ausbeute von 92% der Theorie.

## Beispiel 3

In der Apparatur des Beispiels 1 werden im Verlauf von 50 Minuten 453 g (3,0 Mol) (2-Cyanäthyl)-isocyaniddichlorid in Gegenwart von 900 g (12,67 Mol) Chlor bei 260°C verdampft und über die auf 350°C beheizte Brücke durch das Strömungsrohr geleitet. Zur besseren Wärmeabfuhr wird im das Strömungsrohr konaxial ein unten zugeschmolzenes Glasrohr von etwa 19 mm Durchmesser

eingebracht, so daß das Katalysatorbett in seiner gesemten Länge ringförmig gestaltet ist. Das Volumen das 5 — 6 mm breiten Ringes beträgt etwa 132 ml. Durch den Außenmantel des Strömungsrohres wird Inertgas in dem Maße geleitet, daß sich die Kontakttemperatur zwischen 380 und 400°C hält.

Aufarbeitung analog Beispiel 1 liefert 625 g Kondensat mit einem gaschromatographisch ermittelten Gehalt von 94,2% (entsprechend 588 g) Tetrachlorpyrimidin; das entspricht einer Ausbeute von 90% der Theorie.

Herstellung des Ausgangsproduktes (2-Cyanäthyl)-isocyaniddichlorid:

A) mit Thionylchlorid / Sulfurylchlorid:

In eine gerührte Mischung aus 200 ml (2,48 Mol) Sulfurylchlorid und 1200 ml (16,5 Mol) Thionylchlorid wurden bei 22°C ohne Kühlung im Verlauf einer halben Stunde 98 g (1,0 Mol) (2-Cyanäthyl)-formamid getropft, wobei die Innentemperatur allmählich auf 34°C anstieg; gleichzeitung wurde ein Niederschlago gebildet. Es wurde noch 20 Minuten nachgerührt, wobei die Temperatur auf 30°C sank. Anschließend wurde 2 Stunden bei einer Ölbadtemperatur von 90°C unter Rückfluß gerührt. Bei 50 — 55°C ging der Niederschlag unter Gasentwicklung in Lösung. Am Ende des zweistündigen Erhitzens ist die Rückflußtemperatur bis auf 70°C angestiegen. Nach Abziehen von $SOCl_2$ im Vakuum wurden bei $Kp_{13}$ 107°C 138 g (2-Cyanäthyl)-isocyaniddichlorid in einer gaschromatographisch ermittelten Reinheit von 97,7% (entsprechend 89% der Theorie) erhalten.

B) mit Thionychlorid / Chlor

In einer 4-Liter-Rührapparatur werden 2200 g (18,48 Mol) Thionylchlorid vorgelegt und bei 55°C im Verlauf von 4 Stunden gleichzeitig 453 g (4,62 Mol) (2-Cyanäthyl)-formamid zugetropft und 365 g (5,14 Mol) Chlor eingeleitet. Nach etwa 30 minütigem Nachrühren bei 55 — 60°C ist die Gasentwicklung weitgehend beendet. Die Aufarbeitung erfolgt durch Destillation. Man erhält bei $Kp_{12}$ 100 — 110°C 633 g (2-Cyanäthyl)-isocyaniddichlorid in einer gaschromatographisch ermittelten Reinheit von 95% (entsprechend 95% der Theorie).

**Patentansprüche:**

1. Verfahren zur Herstellung von Tetrachlorpyrimidin, dadurch gekennzeichnet, daß man 2-Cyanäthylisocyaniddichlorid in der Gasphase in Temperaturbereich von 200 — 650°C mit Chlor umsetzt.
2. Verfahren nach Anspruch 1, dadurch gekennzeichnet, daß man mit 3 Mol Chlor umsetzt.
3. Verfahren nach Ansprüchen 1 und 2, dadurch gekennzeichnet, daß man die Umsetzung in Gegenwart von Chlorierungs-Katalysatoren, insbesondere Aktivkohle durchführt.
4. Verfahren nach Ansprüchen 1 — 3, dadurch gekennzeichnet, daß man die Umsetzung im Bereich von 220 — 500°C durchführt.

**Claims**

1. Process for the preparation of tetrachloropyrimidine, characterised in that 2-cyanoethyl isocyanide dichloride is reacted with chlorine in the gas phase in the temperature range from 200—650°C.
2. Process according to Claim 1, characterised in that the reaction is carried out with 3 mols of chlorine.
3. Process according to Claims 1 and 2, characterised in that the reaction is carried out in the presence of chlorination catalysts, in particular active charcoal.
4. Process according to Claims 1—3, characterised in that the reaction is carried out in the range from 200—500°C.

**Revendications**

1. Procédé pour la préparation de tétrachloropyrimidine, caractérisé en ce que l'on fait réagir le dichlorure d'isocyanure de 2-cyanoéthyle avec le chlore en phase gazeuse dans un domaine de températures de 200—650°C.
2. Procédé selon la revendication 1, caractérisé en ce que l'on fait réagir 3 moles de chlore.
3. Procédé selon les revendications 1 et 2, caracterisé en ce que l'on effectue la réaction en présence de catalyseurs de chloruration, en particulier de charbon actif.
4. Procédé selon les revendications 1, 2 et 3, caractérisé en ce que l'on effectue la réaction dans un intervalle de 200—550°C.